# EUROPEAN PATENT APPLICATION

(11) **EP 2 957 294 A1**
(43) Date of publication of application: **23.12.2015**
(21) Application number: 14002137.9
(22) Date of filing: 20.06.2014
(51) Int. Cl.: A61K 38/19

(54) **GCSF for use in the treatment of neurogenic immune suppression and/or prevention of related medical complications**

(71) Applicant: Fraunhofer-Gesellschaft zur Förderung der angewandten Forschung e.V., 80686 München (DE)
(72) Inventor: Wagner, Dr. Daniel-Christoph, 04275 Leipzig (DE); Pösel, Dr. Claudia, 04229 Leipzig (DE); Weise, Dr. Gesa, 04105 Leipzig (DE)
(74) Representative: Teschemacher, Andrea

(57) **Abstract**

The present invention relates to Granulocyte colony-stimulating factor (GCSF) for use in the treatment of neurogenic immune depression syndrome, for use in the prevention of mortality after brain injury and for use in the prevention of brain inflammation after brain injury.

## Description

The present invention relates to Granulocyte colony-stimulating factor (GCSF) for use in the treatment of neurogenic immune depression syndrome, for use in the prevention of mortality after brain injury and for use in the prevention of brain inflammation after brain injury.

Acute central nervous system lesions occurring in events such as stroke, central hemorrhage or traumatic brain injury or spinal cord injury result in the development of rapidly occurring and long-lasting systemic immune depression (Prass et al., 2003, PMID 12939340). In case of a stroke for example, a massive atrophy of the secondary lymphatic organs develops which is inter alia caused by sympathetic nervous system-mediated apoptosis of immune cells. A significant decrease of the number of leukocytes in the blood is also observed. Furthermore, the capability of many inflammatory cells is impaired after a stroke. Indeed, bacterial infections due to impaired immune response are the leading cause of mortality after stroke and they are also associated with decreased neurological recovery of patients (Meisel C, et al., 2005, PMID: 16163382). The effective pathophysiological mechanism of neurogenic immune depression has been understood only recently. So far the occurrence of infection after brain damage has been attributed to hospitality and primary neurological symptoms such as difficulties in swallowing and urination. In the state of the art, it was discussed that neurogenic immune depression is an evolutionary protection mechanism protecting the damaged brain from excessive inflammatory reactions. The recovery of immune competence e.g. mediated by GCSF could therefore have deleterious effects in the brain in addition to the beneficial effects. Up to now, neurogenic immune depression is only subject to preventive or symptomatic approaches (antibiotics), causal approaches have not been followed, let alone clinically implemented.

Surprisingly, the present inventors have now found that Granulocyte colony-stimulating factor (GCSF) can by used in order to treat neurogenic immune depression and prevent mortality and brain inflammation after brain injury. Before the present invention, GCSF has been clinically tested for its use in stroke and brain trauma, but only neuro-protective (brain cell maintaining) and regenerative effects in the brain were subject of the investigation. In clinical trials (Phase II), effectiveness of GCSF in the treatment of stroke could not be confirmed (Ringelstein et al. 2013, PMID: 23963331). Therefore, pharmaceutical companies stopped their research in this field.

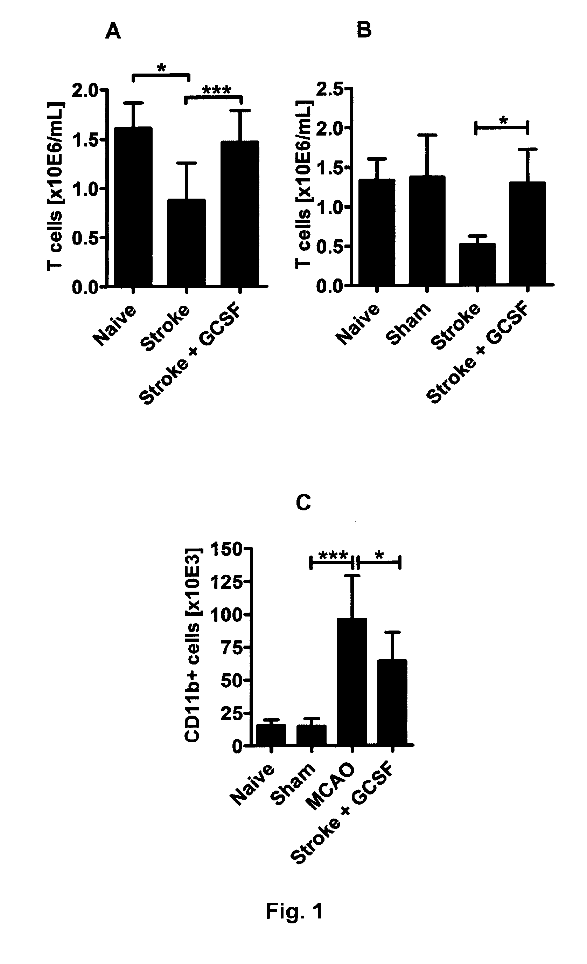

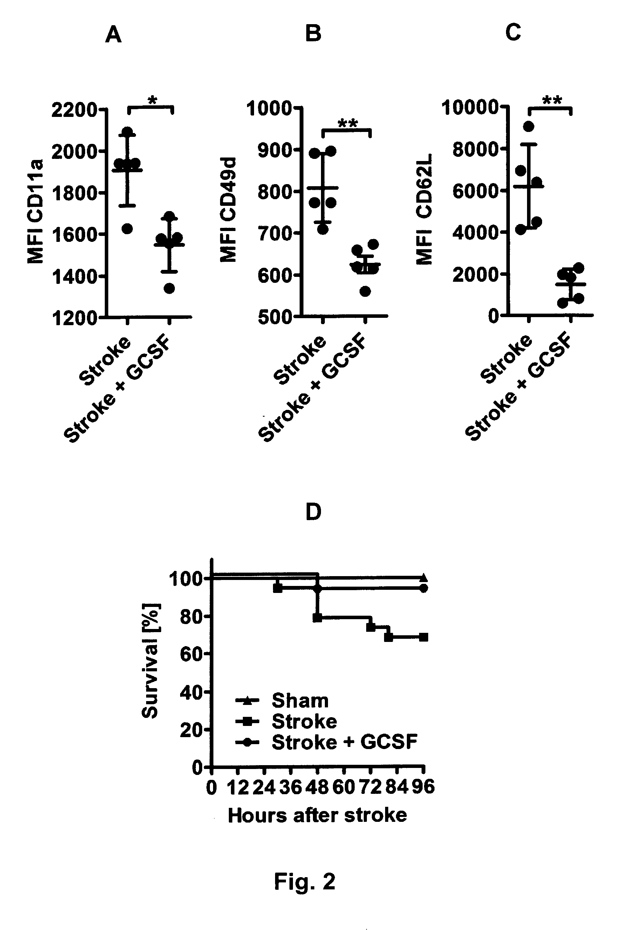

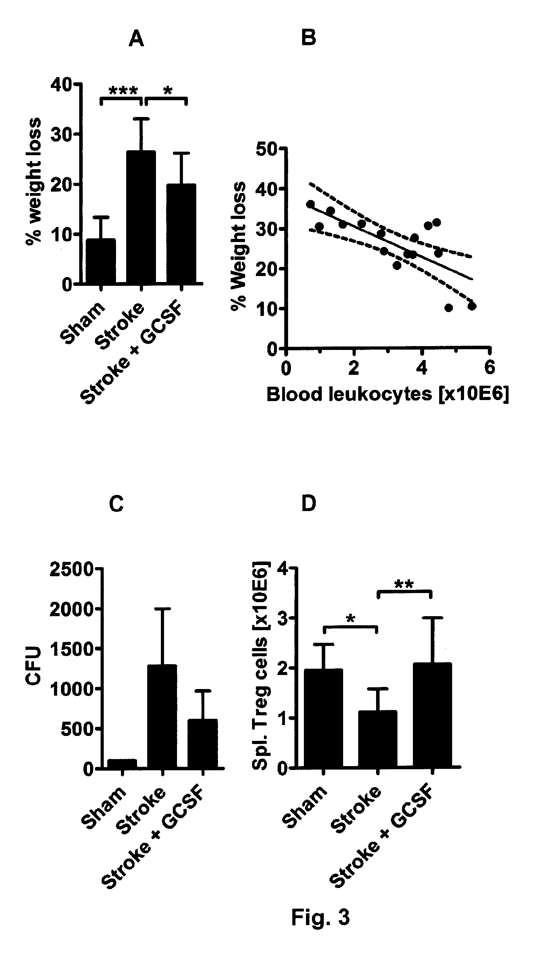

In contrast to the previous studies, the present inventors have now detected in a new stroke study with mice that the treatment with Granulocyte colony-stimulating factor (GCSF) leads to an almost complete recovery of leukocyte numbers and thus reverts neurogenic immune depression (Figure 1A). This finding was confirmed with a second species (rat) (Figure 1 B). Interestingly, the inventors have noticed that inflammatory reactions in the brain decreased during the treatment with GCSF (Figure 2A-C) because of the GCSF-mediated down-regulation of cell surface proteins (adhesion molecules such as CD11a, CD49 and CD62L), which are responsible for the infiltration of inflammatory cells in the ischemic tissue. This results therefore in a recovery in immune competence in addition to the protection of the brain tissue from further inflammation. Accordingly, the immune modulation results in a significant increased survival of animals, which have been treated with GCSF (Figure 2D). A further relevant end point in the stroke animal model is body weight. It is assumed that stroke mediated mortality and infection is preceded by a significant loss of body weight. Inventors observed that GCSF treatment leads to a significant reduction in loss of body weight (Figure 3A). This effect is most probable due to the recovery of peripheral immune competence (Figure 3B). Furthermore, inventors found that the load of anaerobic germs in the lungs of GCSF-treated animals was reduced (Figure 3C). Furthermore, the inventor's data suggest that GCSF does not only recover immune competence but does also mediate tolerance-inducing activities. Neurogenic immune depression does also affect tolerance-inducing inflammatory cells, an effect being fully antagonized by GCSF (Figure 3D). Immune tolerance is a clinically relevant aspect of stroke as the immune system is suddenly confronted with a multitude of brain antigens (from the impaired brain issue). Inadequate tolerance may lead to sequel and impaired healing processes (Becker et al. 2011; PMID: 21799171).

Accordingly, the present invention relates to Granulocyte colony-stimulating factor (GCSF) for use in the treatment of neurogenic immune depression syndrome.

Granulocyte colony-stimulating factor (GCSF or G-CSF), also known as colony-stimulating factor 3 (CSF 3), is a glycoprotein. Functionally, it is a cytokine and hormone, a type of colony-stimulating factor, and is produced by a number of different tissues. GCSF stimulates the bone marrow to produce granulocytes and stem cells and release them into the bloodstream and stimulates the survival, proliferation, differentiation, and function of neutrophil precursors and mature neutrophils.

The natural human glycoprotein exists in two forms, a 174- and 177-amino-acid-long protein. The more-abundant and more-active 174-amino acid form has been used in the development of pharmaceutical products by recombinant DNA technology.

In therapy (oncology and hematology), a recombinant form of GCSF is used. GCSF was first marketed by Amgen with the brand name Neupogen. Several bio-generic versions are now also available in markets such as Europe and Australia. The recombinant human GCSF synthesised in an *E. coli* expression system is called filgrastim. The structure of filgrastim differs slightly from the structure of the natural glycoprotein. Most published studies have used filgrastim. Filgrastim (Neupogen) and PEG-filgrastim (Neulasta) are two commercially-available forms of rhGCSF (recombinant human GCSF). The PEG (polyethylene glycol) form has a much longer half-life, reducing the necessity of daily injections. Another form of recombinant human GCSF called lenograstim is synthesised in Chinese Hamster Ovary cells (CHO cells). As this is a mammalian cell expression system, lenograstim is indistinguishable from the 174-amino acid natural human GCSF. So far, no clinical or therapeutic consequences of the differences between filgrastim and lenograstim have yet been identified. It is emphasized that all these forms of GCSF are encompassed by the present invention.

As detailed above, various forms of GCSF exist and are therapeutically used. This includes GCSF from different species and with different modifications. With respect to the species, human GCSF is preferred for the medical use in humans. GCSF may be modified, e.g. by different posttranslational modifications depending on the expression system used or chemically modified for other purposes, such as for decreasing the clearance rate, improving in stability or abolishing antigenicity of the proteins. The modifications include modification with polyethylene glycol (see e.g. US 6,166,183). GCSF according to the present invention includes modified or unmodified proteins which may include one or more amino-acid changes (deletion, addition, insertion or replacement) as long as such changes will not cause any disadvantageous non-similarity in function to a naturally-occurring GCSF. It is more preferable to use the human GCSF substantially having the amino acid sequence of the naturally-occurring human GCSF, in which no, one, two, three, four or five lysine, aspartic acid or glutamic acid residue(s) is/are included.

In case of PEGylation, the molecular weight of the polyethylene glycol used in the present invention is not restricted to any particular range, being, however, normally of from 500-20,000 and preferably of from 4,000-10,000, which is usually bound to GCSF as known to the person skilled in the art.

The modified GCSF, particularly human GCSF, has essentially the same biological activity as an unmodified intact G-CSF and may accordingly be used in the same application as that. The modified G-CSF has an activity to stimulate the bone marrow to produce granulocytes and stem cells and release them into the bloodstream and particularly to revert neurogenic immune depression syndrome. The later may be assessed as known to the person skilled in the art and/or detailed in the Examples, particularly by determining the number of certain blood cells, survival and/or weight loss caused by neurogenic immune depression. "Essentially the same biological activity" means that the biological activity of the modified GCSF (see above) is reduced by at most 30 %, 25 % or 20%, preferably at most 10 %, especially at most 5 %, relative to the biological activity of the unmodified GCSF.

In the present invention GCSF may be comprised in a pharmaceutical composition. The pharmaceutical composition of the present invention may be formulated in any suitable manner. The pharmaceutical composition of the present invention may further encompass pharmaceutically acceptable carriers and/or excipients. The pharmaceutically acceptable carriers and/or excipients useful in this invention are conventional and may include buffers, stabilizers, diluents, preservatives, and solubilizers. Remington's Pharmaceutical Sciences, by E. W. Martin, Mack Publishing Co., Easton, PA, 15th Edition (1975), describes compositions and formulations suitable for pharmaceutical delivery of the extracts disclosed herein. Depending on the type of application, the GCSF concentration will vary in the form of use.

In general, the nature of the carrier or excipients will depend on the particular mode of administration being employed. For instance, parenteral formulations usually comprise injectable fluids that include pharmaceutically and physiologically acceptable fluids such as water, physiological saline, balanced salt solutions, aqueous dextrose, glycerol or the like as a vehicle. For solid compositions (e. g. powder, pill, tablet, or capsule forms), conventional non-toxic solid carriers can include, for example, pharmaceutical grades of mannitol, lactose, starch, or magnesium stearate. In addition to biologically neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate. Generally, an appropriate amount of a pharmaceutically acceptable salt is used in the carrier to render the formulation isotonic. Examples of the carrier include but are not limited to saline, Ringer's solution and dextrose solution. Preferably, acceptable excipients, carriers, or stabilisers are preferably non-toxic at the dosages and concentrations employed, including buffers such as citrate, phosphate, and other organic acids; salt-forming counter-ions, e.g. sodium and potassium; low molecular weight (> 10 amino acid residues) polypeptides; proteins, e.g. serum albumin, or gelatine; hydrophilic polymers, e.g. polyvinylpyrrolidone; amino acids such as histidine, glutamine, lysine, asparagine, arginine, or glycine; carbohydrates including glucose, mannose, or dextrins; monosaccharides; disaccharides; other sugars, e.g. sucrose, mannitol, trehalose or sorbitol; chelating agents, e.g. EDTA; non-ionic surfactants, e.g. Tween, Pluronics or polyethylene glycol; antioxidants including methionine, ascorbic acid and tocopherol; and/or preservatives, e.g. octadecyldimethylbenzyl ammonium chloride; hexamethonium chloride; benzalkonium chloride, benzethonium chloride; phenol, butyl or benzyl alcohol; alkyl parabens, e.g. methyl or propyl paraben; catechol; resorcinol; cyclohexanol; 3-pentanol; and m-cresol.

The above pharmaceuticals may be administered in any suiatbel manner, including subcutaneously, intramuscularly, intravenously or orally, depending on a purpose of treatment. A dose may be also based on the kind and condition of the disorder of a patient to be treated, being normally between 0.1 µg and 5 mg by injection and between 0.1 mg and 5 g in an oral administration for an adult. However, GCSF is usually given as subcutaneous or intravenous injection.

It has recently become clear that CNS injury (or brain injury) as well as inflammation significantly increases susceptibility to infection by brain-specific mechanisms: CNS injury induces a disturbance of the normally well balanced interplay between the immune system and the CNS. As a result, CNS injury or inflammation leads to secondary immunodeficiency - referred to as neurogenic immune depression syndrome (neurogenic IDS) or CNS injury-induced immunodepression (CIDS) - and infection. Therefore, neurogenic immune depression syndrome (neurogenic IDS) relates to a medical condition in which balanced interplay between the central nervous system and the immune system is impaired and in which the immune system is down regulated. Neurogenic immune depression syndrome includes deactivation of adaptive and innate immunity by over-activating the afferent arm of the neuro-inflammatory reflex that normally protects the body from exaggerated inflammatory responses. Neurogenic immune depression syndrome is mediated by action potentials running along the vagus nerve that ultimately triggers acetyl choline (ACh) secretion from splenic T cells. ACh in turn interacts with the alpha7 subunit of nicotinic acetylcholine receptors on splenic macrophages that down-regulate key pro-inflammatory mediators such as tumor necrosis factor alpha (TNFα) that causes a down-regulation of immune cell activity.

Treatment or treating is the attempted remediation of a health problem, usually following a diagnosis. A treatment treats a problem, and may lead to its cure, but treatments often ameliorate a problem only for as long as the treatment is continued, especially in chronic diseases. Cures are a subset of treatments that reverse illnesses completely or end medical problems permanently. Prevention or preventing is a way to avoid an injury, sickness, death or disease in the first place, and generally it will not help someone who is already ill (though there are exceptions). A treatment or cure is applied after a medical problem has already started, whereas prevention is applied before the above medical problem is detectable. The treatment or prevention may be in any subject, particularly a mammal such as cat, dog, rat, mouse, cow, horse, rabbit, or primate, especially in a human.

In a preferred embodiment the neurogenic immune depression syndrome is characterized by a significant reduction of the number T cells, particularly T helper cells and/or cytotoxic T cells, in the patient's blood relative to a control or reference value.

T cells or T lymphocytes are a type of lymphocyte that play a central role in cell-mediated immunity. They can be distinguished from other lymphocytes, such as B cells and natural killer cells (NK cells), by the presence of a T-cell receptor (TCR) on the cell surface. They are called T cells because they mature in the thymus. There are several subsets of T cells, each with a distinct function.

T helper cells are a type of T cells that play an important role in the immune system, particularly in the adaptive immune system. They help the activity of other immune cells by releasing T cell cytokines. They are essential in B cell antibody class switching, in the activation and growth of cytotoxic T cells, and in maximizing bactericidal activity of phagocytes such as macrophages.

Cytotoxic T cells (also known cytotoxic T lymphocytes, CTL, T-killer cells, cytolytic T cells, CD8+ T-cells or killer T cells) are T lymphocytes that kill cancer cells, cells that are infected (particularly with viruses), or cells that are damaged in other ways.

The number of particular cells in the blood is often an indicator of disease. It is usually determined as absolute number of the cells per volume (e.g. 0.5 x 10⁶/ml or 4.0 x 10E3/µl). The number of specific cells in the blood may be determined by a blood count, also known as blood cell count or blood exam. It is a blood panel usually requested by a doctor or other medical professional that gives information about the cells in a patient's blood. A scientist or lab technician performs the requested testing and provides the requesting medical professional with the results of the blood count. Blood counts of various types have been used for clinical purposes since the 19th century. Automated equipment to carry out complete blood counts was developed in the 1950s and 1960s. Usually flow cytometry is used for determining the number of these diverse cells in the blood. Abnormally high or low counts may indicate the presence of many forms of disease, and hence blood counts are amongst the most commonly performed blood tests in medicine, as they can provide an overview of a patient's general health status.

A significant reduction of the number of cells of a particular type in the patient's blood (or expression of a protein in a cell) relative to a control or reference value may be detected by any suitable means. The person skilled in the art knows statistical procedures to assess whether two values are significantly different from each other such as Student's t-test, ANOVA, Bonferroni's post hoc test or chisquare tests. Based on this, a threshold may be determined by the skilled person, which will evidently depend from the method used. If a value is at or under that threshold, the value is to be regarded as reduced or decreased relative to the control. It is evident for the skilled person that any background signal has to be subtracted when analyzing the data. In specific embodiments, the reduction is at least about 10 % relative to the control. In other embodiments, the decrease is at least 20 %, 30 %, 40 %, 50 %, 60 % or even 75 % or more.

The control or reference value may be a sample from a healthy subject or determined at a group of healthy subjects. Alternatively, it may be a known and/or pre-determined reference value. Particularly for blood analysis reference values are frequently used in the medical field. Furthermore, the skilled person knows how to select a suitable control.

Additionally, neurogenic immune depression syndrome may be further characterized by a significant reduction of the number of B cells, NK cells and/or monocytes, and/or a significant reduction of HLA-DR expression on monocytes in the patient's blood relative to a control or reference value.

B cells or B lymphocytes are a type of lymphocyte in the humoral immunity of the adaptive immune system. B cells can be distinguished from other lymphocytes, such as T cells and natural killer cells (NK cells), by the presence of a protein on the B cells outer surface known as a B cell receptor. This specialized receptor protein allows a B cell to bind to a specific antigen. The principal functions of B cells are to make antibodies against antigens, to perform the role of antigen-presenting cells (APCs), and to develop into memory B cells after activation by antigen interaction.

Natural Killer Cells (or NK cells) are a type of cytotoxic lymphocyte critical to the innate immune system. The role NK cells play is analogous to that of cytotoxic T cells in the vertebrate adaptive immune response. NK cells provide rapid responses to virally infected cells and respond to tumor formation, acting at around 3 days after infection. Typically immune cells detect MHC presented on infected cell surfaces, triggering cytokine release, causing lysis or apoptosis. NK cells are unique, however, as they have the ability to recognize stressed cells in the absence of antibodies and MHC, allowing for a much faster immune reaction. They were named "natural killers" because of the initial notion that they do not require activation in order to kill cells that are missing "self" markers of major histocompatibility complex (MHC) class 1. This role is especially important because harmful cells that are missing MHC 1 markers cannot be detected and destroyed by other immune cells, such as T cells.

Monocytes are a type of leukocytes. They are also part of the innate immune system of vertebrates including mammals. They are amoeboid in shape, having clear cytoplasm. Monocytes have bean-shaped nuclei that are unilobar, which makes them one of the types of mononuclear leukocytes (agranulocytes). Monocytes constitute 2% to 10% of all leukocytes in the human body. They play multiple roles in immune function including replenishing resident macrophages under normal states. In response to inflammation signals, monocytes can move quickly (approx. 8-12 hours) to sites of infection in the tissues and divide/differentiate into macrophages and dendritic cells to elicit an immune response. Monocytes may be identified in stained smears by their large kidney shaped or notched nucleus.

HLA-DR is a MHC class II cell surface receptor encoded by the human leukocyte antigen complex on chromosome 6 region 6p21.31. The complex of HLA-DR and its ligand, a peptide of 9 amino acids in length or longer, constitutes a ligand for the T-cell receptor (TCR). HLA (human leukocyte antigens) were originally defined as cell surface antigens that mediate graft-versus-host disease, which resulted in the rejection of tissue transplants in HLA-mismatched donors. Identification of these antigens has led to greater success and longevity in organ transplant. HLA-DR molecules are regulated in response to signaling. In case of immune depression syndrome its expression on monocytes is down regulated. Monocytic HLA-DR expression may be determined as known to the person skilled in the art (see e.g. Docke et al., 2005, Clin Chem 51(12): 2341-2347).

In a preferred embodiment of the present invention the neurogenic immune depression syndrome is due to a CNS or brain injury or inflammation, particularly due to stroke, traumatic brain injury, spinal cord injury or inflammation. CNS or brain injury is CNS or brain damage caused by events after birth. CNS or brain injury can result in cognitive, physical, emotional, or behavioural impairments that lead to permanent or temporary changes in functioning. These impairments result from either traumatic brain injury (e.g. physical trauma due to accidents, assaults, neurosurgery, head injury etc.) or nontraumatic injury derived from either an internal or external source (e.g. stroke, brain tumours, infection, poisoning, hypoxia, ischemia, encephalopathy or substance abuse). CNS or brain injury does usually not include damage to the brain resulting from neurodegenerative disorders.

The pharmaceutical composition for use of the present invention may be manufactured in any suitable manner. Particularly, the pharmaceutical composition may be manufactured for oral, nasal, rectal, parenteral, topic, vaginal, subcutaneous, intracutaneous, intramuscular, intraarterial, intravenous or intraperitoneal administration. However, subcutaneous or intravenous injection is preferred. GCSF is usually administered by an injection just under the skin in areas such as the abdomen below the naval, upper outer arms, and upper outer thighs. It is possible to self-administer G-CSF depending on the age of the patient. The injection is not usually very painful but, occasionally, a stinging sensation may be experienced for a short period of time. In case of higher dosages, intravenous injection (optionally continuous) may be required. Moreover, GCSF may be used in the treatment or prevention in any subject, particularly a mammal such as cat, dog, rat, mouse, cow, horse, rabbit, or primate. In a preferred embodiment of the present invention, GCSF is used to be administered to a human patient. GCSF can be administered to a patient in any suitable regimen, e.g. as a daily single dose for one to two or several weeks or until neurogenic immune depression does not re-occur. Alternatively, GCSF may be given every second day or even with longer interruption or 2 or more times a day. Depending on the individual patient, maintenance therapy may be required for a loner time including months or even years.

The dose of GCSF may vary widely. Sometimes certain patients may need very high doses, even up to 500 µg per kg body weight and day and others will require very low doses, as low as 0.01 µg/kg/day. There are many factors taken into consideration when deciding a dose of drug - including age of the patient, weight, sex, ethnicity, liver and kidney function and whether the patient smokes. Other medicines may also affect the drug dose. In a preferred embodiment of the present invention, GCSF is to be administered in a dose of from 10 to 1000 µg/(kg body weight (BW)) daily, particularly 20 to 500 µg/kg BW daily, 50 to 300 µg/kg BW daily, more particularly 75 to 200 µg/kg BW daily, especially 100 to 150 µg/kg BW daily. If the molecular weight of GCSF should be increased due to modifications (e.g. PEGYlation), the above values have to be adapted accordingly. The molecular weight of unmodified GCSF is about 20 kDa.

In a preferred embodiment of the present invention, GCSF is to be administered from day 2 or later after the event causing neurogenic immune suppression syndrome, particularly from day 2 after brain injury, especially stroke, traumatic brain injury or spinal cord injury. Several studies suggest that GSCF on day 1 after stroke may worsen clinical symptoms by increased inflammation in brain tissue. This clinically relevant finding was made possible by recalculation of appropriate doses in animal studies (Wagner et al. 2014; PMID: 24407949). Consequently, it could be advisable to start therapy with GCSF later than in the above clinical trials

(Ringelstein et al. 2013; PMID: 23963331). Accordingly, in preferred embodiment treatment with GCSF should started on day 2 or later after the event. This may from day 3, 4, 5, 6 or even later. However, start of GSCF treatment on day 2 after the event is highly preferred.

In a preferred embodiment, GCSF is to be administered to a patient after neurogenic immune depression syndrome had been diagnosed in a patient. Preferably, the diagnosis of neurogenic immune depression is by blood count, especially by detecting (i) a significant reduction of the number of T cells, particularly T helper cells and/or cytotoxic T cells, in the patient's blood relative to a control or reference value and (ii) optionally a significant reduction of the number of B cells, NK cells, monocytes and/or monocyte HLA-DR expression in the patient's blood relative to a control or reference value. Further details on these blood components and their determination are given above. The effectiveness on the GCSF administration may be followed by subsequent blood count or cell number determinations, as detailed above.

In a further aspect, the present invention relates to GCSF for use in the prevention of mortality after brain injury.

Prevention or preventing refers to measures taken to prevent diseases or death, rather than curing them or treating their symptoms. The term contrasts in method with curative and palliative medicine. In the context of the present invention prevention of mortality means that the likelihood of death as a consequence of brain injury is reduced. This means that in a population of patients treated with GCSF after brain injury the number of patients who pass away is reduced relative to that in a control population of untreated patients after brain injury.

In still a further aspect, the present invention relates to GCSF for use in the prevention of brain inflammation after brain injury.

Prevention or preventing is as defined above. In the context of this aspect prevention of brain inflammation means that the likelihood of brain inflammation as a consequence of brain injury is reduced. This means that in a population of patients treated with GCSF after brain injury the number of patients who undergo brain inflammation is reduced relative to that in a control population of untreated patients after brain injury.

In a preferred embodiment, the patient to be treated with GCSF in order to prevent mortality or brain inflammation suffers from neurogenic immune depression syndrome (as specified above). Additionally, the GCSF for use may be further characterized as detailed in the context of GCSF's use in the treatment of neurogenic immune depression syndrome.

The disclosure is not limited to the particular methodology, protocols, and reagents described herein because they may vary. Further, the terminology used herein is for the purpose of describing particular embodiments only and is not intended to limit the scope of the present disclosure. As used herein and in the appended claims, the singular forms "a", "an", and "the" include plural reference unless the context clearly dictates otherwise. Similarly, the words "comprise", "contain" and "encompass" are to be interpreted inclusively rather than exclusively.

Unless defined otherwise, all technical and scientific terms and any acronyms used herein have the same meanings as commonly understood by one of ordinary skill in the art in the field of the disclosure. Although any methods and materials similar or equivalent to those described herein can be used in the practice as presented herein, the specific methods, and materials are described herein.

The disclosure is further illustrated by the following examples and figures, although it will be understood that the examples and figures are included merely for purposes of illustration and are not intended to limit the scope of the disclosure unless otherwise specifically indicated.

### FIGURES

Figure 1. (A, B) T cell counts were suppressed in the blood of rats (A, top left) and mice (B, top right) on day 2 after the stroke (MCAO). Treatment with GCSF increased T cell numbers to the level of naïve and/or sham-operated animals.
Similar results were obtained with further sub-populations of immune cells (B-cells, NK-cells, monocytes). (C) MCAO significantly increased the number of CD11 b+ leukocytes (mostly monocytes and macrophages) in the brain, but this effect was partly reversed by GCSF. *p<0.05, ***p<0.001 by one-way ANOVA.
Figure 2. (A-C) As illustrated in Figure 1C, the treatment with GCSF caused a significant decrease in brain inflammation after stroke, albeit circulating leukocyte counts were increased. This is probably due to a significantly reduced expression of adhesion molecules such as CD11 a (A), CD49d (B) and CD62L (C) on circulating inflammatory cells by GCSF. (D) The treatment with GCSF significantly reduced mortality. MCAO: experimental stroke, LD: low dosage, HD: high dosage. *p<0.05, **p<0.01 by one-way ANOVA.
Figure 3. (A-C) Treatment with GCSF reduced the loss of body weight (A), most probably due to the positive effects on neurogenic immune depression (B) and the reduction of the load of anaerobic germs in the lung (C). (D) Regulatory T-cells may dampen adaptive immune reaction against self-antigens. This is of particular relevance with stroke in order to suppress autoimmune reactions against the brain. Treatment with GCSF normalized the number of these important cells in the spleen (spl). *p<0.05, **p<0.01, by t-test.

### EXAMPLE

### Methods

Stroke was induced either by permanent or transient occlusion of the right middle cerebral artery (MCAO) in male spontaneously hypertensive rats or C57BL/6 mice. For permanent MCAO, the right temporal bone and subjacent dura mater were opened and the middle cerebral artery was elevated by a thin hook mounted in a stereotactic frame and occluded by thermocoagulation. For transient MCAO, the common and the external carotid arteries were exposed and ligated. A silicon-rubber coated monofilament was then introduced into the common carotid artery and advanced towards the origin of the middle cerebral artery. 45 minutes later, the filament was removed to allow for cerebral re-perfusion. Animals were randomly assigned to either a control or a treatment group. Animals of the treatment group received rhGCSF (Neupogen, Amgen Thousand Oaks, USA) at 416.25µg per kg bodyweight via intraperitoneal injection directly after and 12h after stroke. Treatment was controlled by glucose (5%). Treatment was continued over at least 4 days at the same dose and frequency.

Two days after stroke onset, animals were sacrificed by CO2 exposition under deep isoflurane anesthesia. Blood was collected via cardiac puncture and mixed with 2mM EDTA. Absolute leukocyte counts were determined by an animal blood cell counter (scil Vet abc, SCIL animal care company GmbH, Viernheim, Germany). Analysis of brain tissue inflammation was performed in tissue single cell suspensions by means of flow cytometry. Briefly, brain tissue was homogenized using a 100µm cell strainer and by digestion with 2U/mL Liberase TL (Roche). Leukocytes were then enriched by density gradient centrifugation using 25% Percoll at 520g for 20 minutes.

For flow cytometry, blood and brain tissue lysates were diluted with 50µl phosphate buffered solution and in incubated with specific fluorophore-coupled monoclonal antibodies against CD45, NK1.1, Ly6G, CD25, CD3, CD4, CD8, B220, CD11c, Ly6C, MHC-II, CD11 b, CD11 a, CD49d, CD62L, Foxp3. Flow cytometry was performed by a researcher that was blinded to the group allocation using a FACS Canto II and analyzed by FlowJo software (Tree Star). Leukocyte sub-populations were determined by specific patterns of marker expression (e.g. CD3 for T cells, CD3/CD4 for T helper cells, Cd3/CD4/CD25/Foxp3 for regulatory T cells) using intensity thresholds that were defined in autofluorescence controls. Expression of adhesion molecules were determined by calculating the median fluorescence intensity (MFI) minus the autofluorescence control.

For gene expression analysis, tissues (spleen, lymph nodes and brain) were removed and shock-frozen in liquid nitrogen. After dissociation and cDNA generation, we used Quantitect Primer assays and SYBR Green (Quigane) for qRT-PCR.

Data was analyzed by Graphpad Prism software. P values of 0.05 or less were considered to be statistically significant. Data were analyzed by t-test or one way ANOVA followed by Bonferroni's post hoc test.

### Results

Treatment of cerebral stroke using GCSF caused a rapid and significant recovery of peripheral and lymphatic immune cell counts that were decreased due to neurogenic immunodepression. This was detectable not only for T cells (Figure 1A and B) but also for T cell subpopulations such as T helper cells (stroke: 5.4 ± 3.2x10E6 versus stroke + GCSF: 13.7 ± 5.6 x10E6, p=0.012 by t-test) cytotoxic T cells (stroke: 4.8 ± 3.0x10E6 versus stroke + GCSF: 13.9 ± 4.0 x10E6, p=0.0018 by t-test) and gamma-delta T cells (stroke: 0.2 ± 0.1x10E6 versus stroke + GCSF: 0.5 ± 0.3 x10E6, p=0.0039 by t-test). Moreover, we observed recovery of B cells (stroke: 13.9 ± 10.3x10E6 versus stroke + GCSF: 57.5 ± 24.6x10E6, p=0.0033 by t-test), NK cells (stroke: 0.4 ± 0.3x10E6 versus stroke + GCSF: 2.2 ± 1.0x10E6, p=0.0022 by t-test) and Ly6c+ inflammatory monocytes (stroke: 0.4 ± 0.3x10E6 versus stroke + GCSF: 1.8 ± 0.8x10E6, p=0.0001 by t-test). Similar results were found in the circulation and in other lymphatic tissue. At the same time, we found that the expression of important soluble mediators of immunity was significantly increased by GCSF treatment (for example IL-1β: 15fold increased expression, p=0.008 by t-test; CCL6: 6fold increased expression by GCSF, p=0.009 by t-test). Stroke is accompanied by a significant inflammatory response in the brain tissue that has the potential to aggravate the initial ischemic damage. Restoration of circulating immune cell counts could hence be positive by preventing infections but detrimental by increased neuroinflammation. Interestingly, we found rather decreased brain inflammation (Figure 1C), likely as a consequence of decreased adhesion molecule expression in circulating immune cells after GCSF treatment (Figure 2A). Importantly, we found that the regulatory arm of the immune system was also activated by GCSF (Figure 3D). The beneficial consequences of GCSF-mediated recovery of neurogenic immunodepression were corroborated by reduced mortality (Figure 2B) and weight loss (Figure 3A) and by a decreased number of anaerobic bacteria in the lungs (Figure 3A).

## Claims

1. Granulocyte colony-stimulating factor (GCSF) for use in the treatment of neurogenic immune depression syndrome.

2. GCSF for use according to claim 1, wherein neurogenic immune depression syndrome is **characterized by** a significant reduction of the number of T cells, particularly T helper cells and/or cytotoxic T cells, in the patient's blood relative to a control or reference value.

3. GCSF for use according to claim 2, wherein neurogenic immune depression syndrome is **characterized by** a significant reduction of the number of B cells, NK cells and/or monocytes, and/or a significant reduction of HLA-DR expression on monocytes in the patient's blood relative to a control or reference value.

4. GCSF for use according to any of claims 1 to 3, wherein neurogenic immune depression syndrome is due to a brain injury.

5. GCSF for use according to any of claims 1 to 4, wherein neurogenic immune depression syndrome is due to stroke, traumatic brain injury, spinal cord injury or inflammation.

6. GCSF for use according to any of claims 1 to 5, wherein GSCF is to be administered to a human patient.

7. GCSF for use according to any of claims 1 to 6, wherein GCSF is to be administered in a dose of from 10 to 1000 µg/(kg body weight (BW)) daily, particularly 20 to 500 µg/kg BW daily, 50 to 300 µg/kg BW daily, more particularly 75 to 200 µg/kg BW daily, especially 100 to 150 µg/kg BW daily.

8. GCSF for use according to any of claims 1 to 7, wherein is to be administered from day 2 after the event causing neurogenic immune suppression syndrome, particularly from day 2 after brain injury, especially stroke, traumatic brain injury or spinal cord injury.

9. GCSF for use according to any of claims 1 to 8, wherein GCSF is to be administered after neurogenic immune depression syndrome had been diagnosed in a patient.

10. GCSF for use according to claim 9, wherein the diagnosis is by blood count, especially by detecting (i) a significant reduction of the number of T cells, particularly T helper cells and/or cytotoxic T cells, in the patient's blood relative to a control or reference value and (ii) optionally a significant reduction the number of B cells, NK cells, monocytes and/or monocyte HLA-DR expression in the patient's blood relative to a control or reference value.

11. GCSF for use in the prevention of mortality after brain injury.

12. GCSF for use in the prevention of brain inflammation after brain injury.

13. GSCF for use of claim 11 or 12, wherein the patient to be treated with GCSF suffers from neurogenic immune depression syndrome and the GCSF for use is further characterized as in any of claims 2 to 10.
